# EUROPEAN PATENT SPECIFICATION

(11) **EP 1 344 768 B1**
(45) Date of publication and mention of the grant of the patent: **06.04.2005**
(21) Application number: 03006019.8
(22) Date of filing: 10.08.2000
(51) Int. Cl.: C07D 487/04, C07C 231/08, C07C 233/43

(54) **Synthesis of substituted pyrazolopyrimidines**
Synthese substituierter Pyrazolopyrimidine
Synthèse de pyrazolopyrimidines substituées

(30) Priority: 10.08.1999 US 148313 P; 10.09.1999 US 148314 P
(43) Date of publication of application: 17.09.2003
(62) Divisional of application: 00955442.9
(73) Proprietor: Neurocrine Biosciences, Inc., San Diego, CA 92121 (US)
(72) Inventor: Gross, Raymond S., San Diego, CA 92064 (US); Wilcoxen, Keith M., San Diego, CA 92122 (US); OGLESBY, Richard Christopher, New York 12156 (US)
(74) Representative: Grünecker, Kinkeldey, Stockmair & Schwanhäusser Anwaltssozietät

(56) References cited:
- EP-A- 0 073 715
- EP-A- 0 263 438
- US-A- 4 654 347
- US-A- 4 900 836
- US-A- 5 538 977

## Description

### TECHNICAL FIELD

This invention is directed to the synthesis of a substituted pyrazolopyrimidine, which compound has utility over a wide range of indications including treatment of insomnia.

### BACKGROUND OF THE INVENTION

U.S. Patent No. 4,521,422 to American Cyanamid Company is directed to certain aryl and heteroaryl[7-(aryl and heteroaryl)-pyrazolo[1,5-a]pyrimidin-3-yl]methanones which are active as anxiolytic, anticonvulsant, sedative-hypnotic and skeletal muscle relaxant agents. Such compounds may generally be classified as "substituted pyrazolopyrimidines" having the following structure (I): 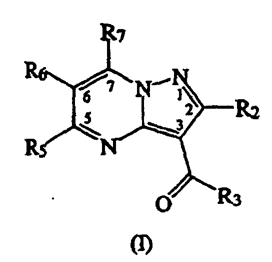 wherein R₂, R₃, R₅, R₆ and R₇ are as defined in U.S. Patent No. 4,521,422 (but under a different numbering scheme for the various R groups).

Compounds of structure (I) are made according to U.S. Patent No. 4,521,422 by reacting an appropriately substituted pyrazole (a) with an appropriately substituted 3-dimethylamino-2-propen-1-one (b) as represented by the following Reaction Scheme A:

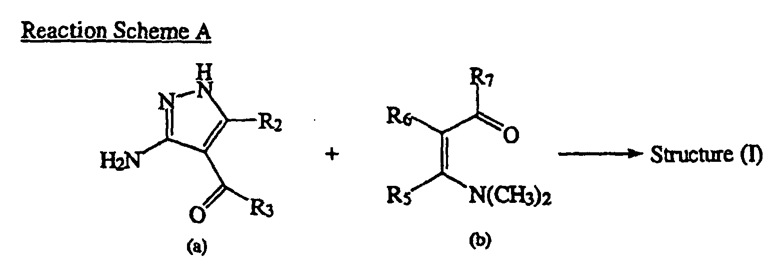

U.S. Patent No. 4,900,836, also to American Cyanamid Company, discloses novel pyrazoles (a) for use in Reaction Scheme A. More specifically, in this patent pyrazoles (a) are made, as represented below in Reaction Scheme B, by reacting appropriately substituted acetonitrile (c) with a dimethylamide dimethyl acetal (d) to yield the corresponding propanenitrile (e), which is then reacted with aminoguanidine nitrile (f) to give the correspondingly substituted pyrazole (a).

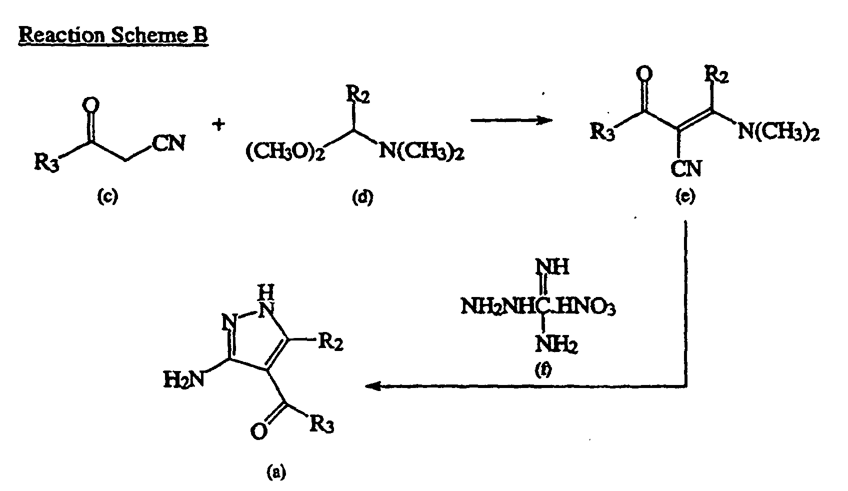

U.S. Patent No. 4,521,422 also discloses the synthesis of substituted pyrazolopyrimidines of structure (I) using starting intermediates other than substituted 3-dimethylamino-2-propen-1-ones (b). For example, as represented by Reaction Scheme C, U.S. Patent No. 4,521,422 teaches that the intermediate alkali metal salts of hydroxymethyleneketones (g) can be acylated by reacting with acid chlorides or anhydrides to give O-acyl derivatives (h), and that neutralization of (g) with certain acids affords hydroxymethylene ketones (i), all of which may be reacted with pyrazole (a) to give compounds of structure (I).

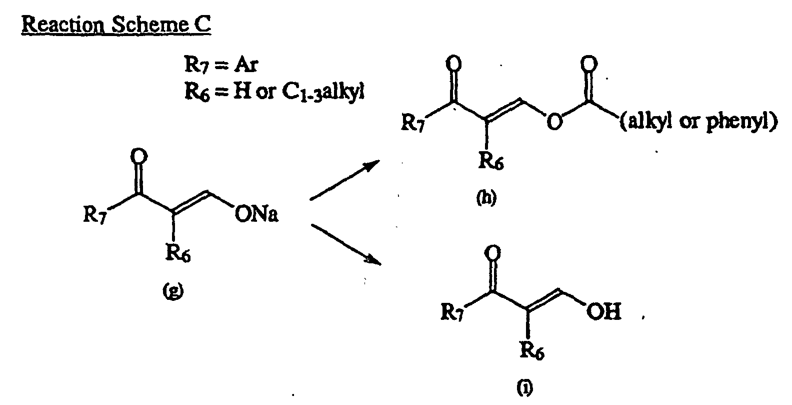

Further, U.S. Patent No. 4,521,422 discloses that hydroxymethyleneketones (i) may be converted to other aldehyde equivalents, such as alkoxymethyleneketones (j), alkylthiomethyleneketones (k) and aminomethyleneketones (1) - as represented by Reaction Scheme D - and reacted with pyrazole (a) to give compounds of structure (I). Other hydroxymethleneketone and derivatives which are chemical equivalents of the same may also be used, such as 2-(dialkylamino)-1-aryl or (heteroatyl)-2 propen-1-ones.

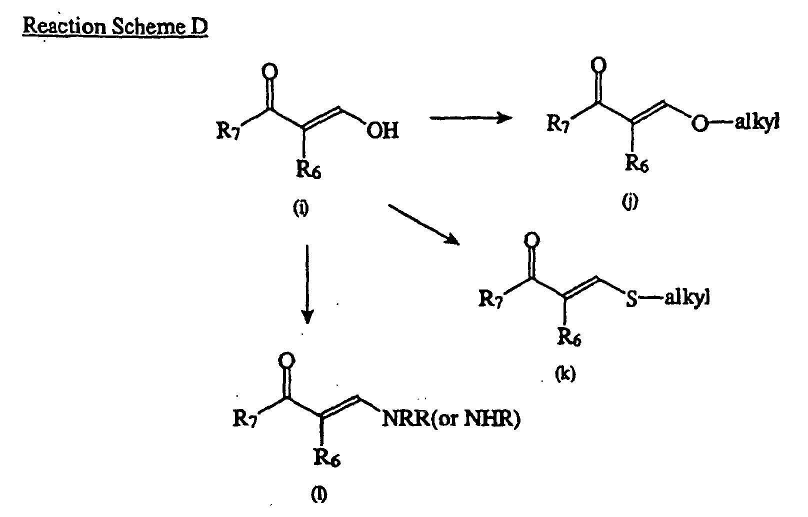

In addition, other United States patents have issued directed to the synthesis of substituted pyrazolopyrimidines. For example, U.S. Patent Nos. 4,654,347 and 4,626,538, both to American Cyanamide, disclose substituted pyrazolopynmidines of structure (I) made by the techniques discussed above, but having different substituents than those of U.S. Patent No. 4,521,422.

While U.S. Patent Nos. 4,521,422 and 4,900,836, among others, teach techniques for the synthesis of compounds of structure (I), such procedures are relatively time consuming, involve numerous steps, and are not particularly economical or efficient for large-scale synthesis. Accordingly, there is a need in the art for improved synthetic techniques which overcome these shortcomings. The present invention fulfills these needs, and provides further related advantages.

### SUMMARY OF THE INVENTION

In brief, the present invention is directed to the synthesis of a substituted pytazolopyrimidine having the following structure (1), which compound has utility over a wide range of indications, including treatment of insomnia.

### DETAILED DESCRIPTION OF THE INVENTION

As mentioned above, the present invention is directed to an improved synthetic route for the synthesis of a substituted pyrazolopyrimidine which is active as anxiolytic, anticonvulsant, sedative-hypnotic and skeletal muscle relaxant agents. This compound is covered by structure (I) above previously disclosed in U.S. Patent No. 4,521,422.

More specifically, the pyrazolopyrimidine of structure (I) is N-methyl-N-(3-{3-[2-thienylcarbonyl]-pyrazolo-[1,5-a]-pyrimidin-7-yl}phenyl)acetamide, which compound has the following structure (hereinafter referred to as "Compound 1" or "1"):

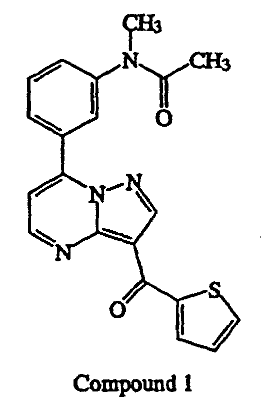

In one aspect of the invention, compound 1 is prepared by the following 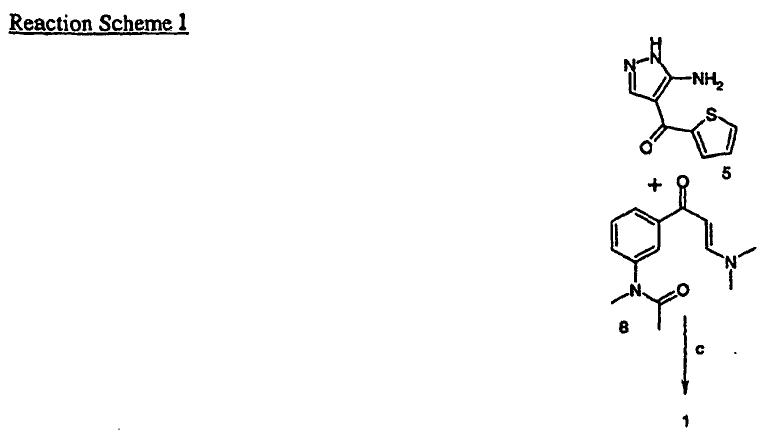 Reagents: (c) HOAc, reflux

In Reaction Scheme 1, pyrazole 5 is cyclized with the known enaminone 8 under the standard conditions providing the desired product, Compound 1.

More particularly, the invention concerns a method for making alkylated enaminone 8, composing the step of alkylating enaminone 8' under phase transfer conditions.

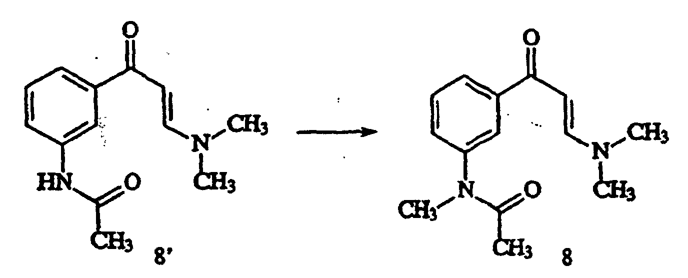

As used herein, "phase transfer conditions" means an organic substrate is dissolved in an organic solvent and the nucleophilic reagent is dissolved in an aqueous phase, a phase transfer catalyst is used to carry the nucleophile from the aqueous phase into the organic phase to react with the substrate. More specifically, phase transfer conditions in the context of this invention may be obtained by utilizing, for example, a phase transfer catalyst (such as quaternary ammonium or phosphonium salts, crown ethers and polyethylene glycol ethers) in a polar organic solvent (such as methylene chloride, benzotrifluoride, or toluene) with an aqueous phase containing a base (such as sodium or potassium hydroxide).

The following example is offered by way of illustration, not limitation.

### EXAMPLE

### FORMATION OF ENAMINONE 8 UNDER PHASE TRANSFER CONDITIONS

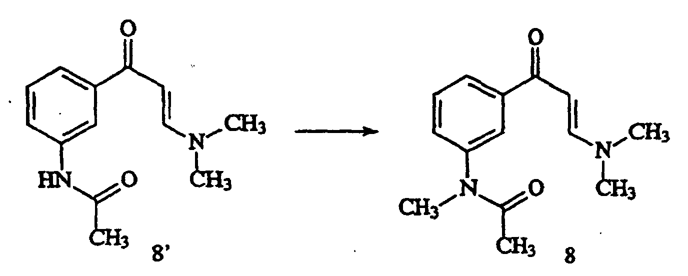

To enaminone 8' (25 g, 107.6 mmol) in benzotrifluoride (92 mL) and dichloromethane (160 mL) was added tetrabutytammonium sulfate (2 g, 5.9 mmol). To the mixture was added of dimethyl sulfate (16 g, 126.7 mmol). To the mixture was added 200 g of 50% aqueous sodium hydroxide solution and the mixture vigorously stirred for 6 hours, keeping the reaction temperature less than 40°C. Upon consumption of starting material, 200 mL of water was carefully added keeping the reaction temperature less than 40°C. The aqueous phase was separated and the organic phase was washed three times with water and dried over magnesium sulfate. The dichloromethane was removed *in vacuo* affording a yellow solid. The solid was filtered, washed with benzotrifluoride and dried *in vacuo* affording alkylated enaminone 8 (19 g, 72% yield).

## Claims

1. A method for making alkylated enaminone. 8, comprising the step of alkylating enaminone 8' under phase transfer conditions

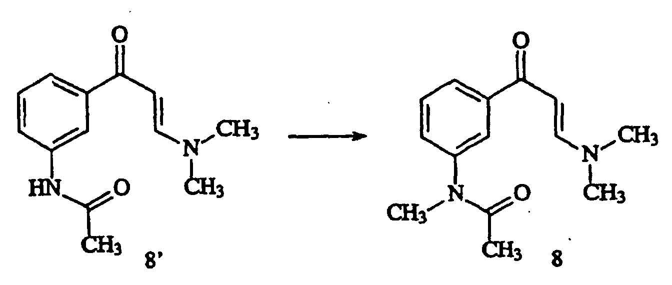

2. The method of claim 1 wherein the phase transfer conditions comprise a phase transfer catalyst in a polar organic solvent with an aqueous phase containing a base.

3. The method of claim 2 wherein the phase transfer catalyst is a quaternary ammonium or phosphonium salt, a crown ether or a polyethylene glycol ether.

4. The method of claim 2 wherein the organic solvent is methylene chloride, benzotrifluoride or toluene.

5. The method of claim 2 wherein the base of aqueous phase is sodium or potassium hydroxide.

6. The method of claim 1, further comprising the step of utilizing enaminone 8 as an intermediate in the synthesis of N-methyl-N-(3-{3-[2-thienylcarbonyl]-pyrazolo-[1,5-a]pyrimidin-7-yl}phenyl)acetamide (Compound 1), wherein the step of utilizing enaminone 8 as an intermediate comprises reacting enaminone 8 with a pyrazole having the structure: 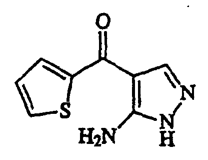 to form Compound 1.

## Patentansprüche

1. Verfahren zum Herstellen von alkyliertem Enaminon 8, umfassend den Schritt des Alkylierens von Enaminon 8' unter Phasentransferbedingungen

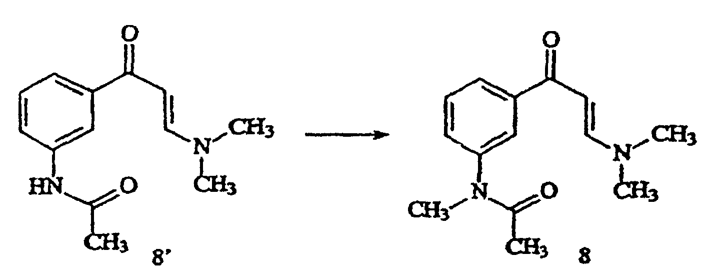

2. Verfahren nach Anspruch 1, wobei die Phasentransferbedingungen einen Phasentransferkatalysator in einem polaren organischen Lösungsmittel mit einer wässrigen Phase, die eine Base enthält, umfassen.

3. Verfahren nach Anspruch 2, wobei der Phasentransferkatalysator ein quaternäres Ammonium- oder Phosphoniumsalz, ein Kronenether oder ein Polyethylenglycolether ist.

4. Verfahren nach Anspruch 2, wobei das organische Lösungsmittel Methylenchlorid, Benzotrifluorid oder Toluol ist.

5. Verfahren nach Anspruch 2, wobei die Base der wässrigen Phase Natrium- oder Kaliumhydroxid ist.

6. Verfahren nach Anspruch 1, außerdem umfassend den Schritt des Verwendens von Enaminon 8 als ein Zwischenprodukt bei der Synthese von N-Methyl-N-(3-{3-[2-thienylcarbonyl]-pyrazolo-[1,5-a]-pyrimidin-7-yl}phenyl)acetamid (Verbindung 1), wobei der Schritt des Verwendens von Enaminon 8 als ein Zwischenprodukt das Umsetzen von Enaminon 8 mit einem Pyrazol mit der Struktur: 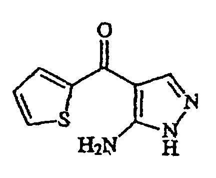 zum Bilden der Verbindung 1 umfasst.

## Revendications

1. Une méthode de formation de l'énaminone 8 alkylée, comprenant l'étape d'alkylation en conditions de transfert de phase de l'énaminone 8'.

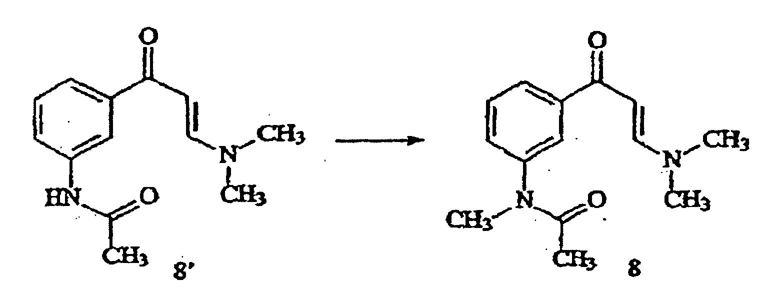

2. La méthode de la revendication 1 dans laquelle la condition de transfert de phase se compose d'une phase de catalyse dans un solvant organique polaire avec une phase aqueuse contenant une base.

3. La méthode de la revendication 2 dans laquelle le transfert de phase de catalyse est un ammonium quaternaire ou un sel de phosphonium, un éther couronne ou un éther de polyéthylène glycol.

4. La méthode de la revendication 2 dans laquelle le solvant organique est du chloride de méthylène, du benzotrifluoride ou du toluène.

5. La méthode de la revendication 2 dans laquelle la base de la phase aqueuse est de l'hydroxide de sodium ou de potassium.

6. la méthode de la revendication 1, qui de plus se compose de l'étape d'utilisation de l'énaminone 8 comme un intermédiaire de la synthèse du N-méthyl-N-(3-{3-[2-thienylcarbonyl]-pyrazolo-[1,5-a]-pyrimidine-7-yl}phényl) acétamide (Composant 1), dans laquelle l'étape d'utilisation de l'énaminone 8, comme un intermédiaire, comprend l'émanione réagissant avec un pyrazole ayant la structure ci-dessous afin de former le composant 1.
